# EUROPEAN PATENT SPECIFICATION

(11) **EP 2 737 316 B1**
(45) Date of publication and mention of the grant of the patent: **16.05.2018**
(21) Application number: 12808036.3
(22) Date of filing: 02.07.2012
(51) Int. Cl.: G01N 33/569, C12Q 1/04, C07K 16/12

(54) **A DIAGNOSTIC KIT FOR THE DETECTION OF EARLY ACUTE LEPTOSPIROSIS**
DIAGNOSEKIT ZUR FRÜHERKENNUNG VON AKUTER LEPTOSPIROSE
KIT DE DIAGNOSTIC POUR LA DÉTECTION PRÉCOCE DE LA LEPTOSPIROSE AIGUË

(30) Priority: 05.07.2011 MY PI2011700101
(43) Date of publication of application: 04.06.2014
(73) Proprietor: Universiti Putra Malaysia (UPM), Selangor (MY)
(72) Inventor: BAHAMAN, Abdul Rani, 43400 Serdang Selangor (MY); SEENICHAMY, Arivudainambi, 43400 Serdang Selangor (MY)
(74) Representative: Krauss, Jan
(86) International application number: PCT/MY2012/000196
(87) International publication number: WO 2013/006034

(56) References cited:
- WO-A1-02/075310
- WO-A2-2004/032599
- US-A1- 2010 015 634
- A CARRIZO ET AL: "Antígenos L. interrogans 129 Identificación de antígenos inmunorreactivos de Leptospira interrogans", REVISTA ARGENTINA DE MICROBIOLOGIA., vol. 41, no. 3, 1 June 2009 (2009-06-01), pages 129-133, XP055162784, ISSN: 0325-7541
- LUO D ET AL: "Protein typing of major outer membrane lipoproteins from Chinese pathogenic Leptospira spp. and characterization of their immunogenicity", VACCINE, ELSEVIER LTD, GB, vol. 28, no. 1, 10 December 2009 (2009-12-10), pages 243-255, XP026789466, ISSN: 0264-410X, DOI: 10.1016/J.VACCINE.2009.09.089 [retrieved on 2009-09-29]
- ARIVUDAINAMBI SEENICHAMY ET AL: "Production and Characterization of a Polyclonal Antibody of Anti-rLipL21-IgG against Leptospira for Early Detection of Acute Leptospirosis", BIOMED RESEARCH INTERNATIONAL, vol. 10, no. 2, 1 January 2014 (2014-01-01), pages 445-8, XP055162747, ISSN: 2314-6133, DOI: 10.1309/BGWQ-P26T-7TR6-VGT3
- CHEEMA, P. ET AL.: 'Detection of pathogenic leptospires in animals by PCR based on lipL21 and lipL32 genes' INDIAN JOURNAL OF EXPERIMENTAL BIOLOGY vol. 45, June 2007, pages 568 - 573, XP055146623
- LUO, D. ET AL.: 'Reconstruction of leptospira interrogans lipL21 gene and characteristics of its expression product' ZHEJIANG DA XUE XUE BAO YI XUE BAN vol. 36, no. 5, September 2007, pages 458 - 464, XP008172912
- LIN, X. ET AL.: 'Identification of immunodominant B- and T- cell combined epitopes in outer membrane lipoproteins LipL32 and LipL21 of Leptospira interrogans' CLINICAL AND VACCINE IMMUNOLOGY vol. 17, no. 5, 17 March 2010, pages 778 - 783, XP055146624
- HE, H. ET AL.: 'Protection of guinea pigs against Leptospira interrogans serovar lai by LipL21 DNA vaccine' CELLULAR AND MOLECULAR IMMUNOLOGY vol. 5, no. 5, October 2008, pages 385 - 391, XP055146630
- CULLEN, P. ET AL.: 'LipL21 is a novel surface-exposed lipoprotein of pathogenic Leptospira species' INFECTION AND IMMUNITY vol. 71, no. 5, May 2003, pages 2414 - 2421, XP055146631
- COLIGAN ET AL: "Current Protocols in Immunology", CURRENT PROTOCOLS IN IMMUNOLOGY, XX, XX, vol. 1, 1 January 1991 (1991-01-01), pages 2.1.1-2.1.22, XP003001177,

## Description

### Field of the Invention

The present invention relates to an *in vitro* diagnostic method for the detection of acute leptospirosis according to the appended claims. The method of the invention allows for a quick, specific and sensitive detection of the disease which is caused by pathogenic *Leptospira.* The described method is based on the immunological detection of the protein LipL21, a membrane protein expressed specifically in pathogenic strains of *Leptospira.* Furthermore, the present invention relates to a diagnostic device that allows to conduct the inventive method in a simple and quick approach. The preferred diagnostic device of the invention is the lateral flow test, which as a hand held assay is easy to use and further allows diagnosis of leptospirosis to be performed as early as the first 5 days of infection. It also disclosed the antibody raised against the LipL21 protein as well as its use in the diagnostic methods and devices. Finally a diagnostic kit is disclosed containing the inventive materials described herein for conducting a diagnosis of leptospirosis.

### Background of the Invention

Leptospirosis or Weil's Disease is a bacterial infection caused by *Leptospira,* which is pathogenic for a wide range of animal species including humans. The pathogen was discovered for the first time in 1886 by Adolf Weil who reported an acute infection with enlargement of spleen, jaundice and nephritis. It occurs in the western countries, like the United States and Canada, specifically in dogs, as well as in tropical countries, for example in Asia. Incident rates range in temperate countries from 0.02 per 100,000 to 10 to 100 in 100,000 in warm/tropical countries. Leptospirosis outbreaks were observed during the wet and warm months in the year, particularly during the monsoon season.

The *Leptospira* organism is transmitted through the urine of an infected animal. The most important primary host of the pathogen are rodents, particularly mice and rats, however, there are also a wide range of animals that are able to carry and transmit the disease. Humans get infected mainly from their pets or when working in close proximity to infected animals, like for example in slaughterhouse. The route of infection in humans is mainly through contact with contaminated water, food or soil.

Usually the disease symptoms begin with typical signs of flu, like fever, chills and headache. When this first phase of the disease resolves, the patient is for a short period of time asymptomatic until the second phase of the disease begins. The second phase is characterized by meningitis, liver damage and renal failure, and can induce severe damage when left untreated. Symptoms in humans usually occur in Day 4 to 14 following infection.Because of this wide range of different symptoms, the disease is often misdiagnosed. Thus, recorded cases of *Leptospira* infection are estimated to be below the actual incident rate.

Leptospirosis is confirmed with serological tests such as the microscopic agglutination test (MAT) and the enzyme-linked immunosorbent assay (ELISA). However, the serological techniques that are used in detecting leptospiral antibodies are very tedious, time consuming and often neither specific nor sensitive. The serological results were difficult to interpret and making them unsuitable for routine testing particularly on large number of samples. These serological methods detect either IgM or IgG antibodies to *Leptospira.*

The detection of leptospires in the form of culture isolation is also not preferable due to the slow growth of the bacterium and therefore provides a limited use in laboratory investigation. Nizamuddin*et al*., (2006) suggested that antigen detection is useful in the diagnosis of acute cases of leptospirosis. However, to date, no diagnostic kit is available to detect antigen(s) in acute leptospiremia, specifically not during the first week of infection.

Available diagnostic tests, which are based on the use of the whole leptospiral cell extracts or semi purified fractions of proteins of the native non-infective form, can detect antibodies against leptospirosis with increased sensitivity and specificity in the late acute phase (Effler *et al*., 2002).

Lateral flow tests (LFT), also known as Lateral Flow Immunochromatographic Assays are simple diagnostic test devices used to detect the presence (or absence) of a target analyte, e.g. a pathogen, in a sample. Most commonly, these tests are used in medicine to provide an easy diagnosis either for use in the clinic or in the laboratory. Often produced in a dipstick format or as a strip, the lateral flow tests are a form of immuno-based assays in which the test sample moves along a solid substrate via capillary force.

After the sample is applied to the test it encounters a coloured reagent which mixes with the sample and transits to the substrate encountering lines which have been pre-treated with an antibody or antigen. Depending upon the analyte's presence in the sample the coloured reagent can become bound at the test line. Lateral Flow Tests can operate as either competitive or sandwich assays.

The diagnostic techniques of immunological assays are mostly based on detecting the antibody or antigen using a variety of methods. Labelled-antigens or antibodies are playing a prominent role in many of the currently available diagnostic kits. The label may consist of an enzyme, colloidal gold, radioisotope or fluorescent probe. The extremely high selectivity and affinity of the host antibody molecules to specific antigens have widely been used for immune detection methods. IgM antibody-based immune assays are used for early detection of many infectious diseases. However, serious infectious diseases like leptospirosis could kill the infected subject before the host could muster a significant immune response and produces enough antibodies to allow detection in the diagnostic method.

WO 2004/032599 discloses LigA and LigB proteins from *Leptospira* for use in the diagnostics of an infection.

US 2010/015634 discloses lateral flow diagnostic testing devices.

WO 02/075310 discloses methods for testing a plurality of different pathogens in a milk sample of a mammal.

A CARRIZO ET AL; REVISTA ARGENTINA DE MICROBIOLOGIA., (2009/06/01), vol. 41, no. 3,, pages 129 - 133, disclose the identification of antigens of *Leptospira interrogans.*

LUO D ET AL, VACCINE, ELSEVIER LTD, GB, vol. 28, no. 1, pages 243 - 255, (2009/09/29), disclose protein typing of the outer membrane of *Leptospira spp.*

COLIGAN ET AL; CURRENT PROTOCOLS IN IMMUNOLOGY , 1 January 1991; disclose standard immune assays such as an ELISA and sandwich ELISA.

In view of the available diagnostic assays for leptospirosis it was the objective of the present invention to provide an improved diagnostic test system which allows for an easy, rapid and specific detection of the pathogenic *Leptospira* organisms. Specifically, the objective of the invention was to provide a diagnostic test that allows for the diagnosis of leptospirosis in the very early stage of infection, specifically in the first 10 days, or even within the first 1 to 4 days of infection.

The above problem is solved in a first aspect by an *in-vitro* method for the detection of the presence of *Leptospira* in a sample, comprising the steps of
a) providing a sample to be analyzed obtained from a subject suspected to suffer from a Leptospira infection between day 1 and day 7 of the infection,
b) detecting the presence of LipL21 in the said sample by use of a polyclonal rabbit anti recombinant-LipL21 (rLipL21) IgG antibody specific for the LipL21 protein,
   wherein the presence of LipL21 in the said sample is indicative for the presence of Leptospira.

In the context of the present invention a sample usable in the methods and devices described herein is a sample obtained from a subject suspected to suffer from a *Leptospira* infection. Such a sample is preferably a biopsy, preferably a kidney biopsy, or a liquid sample, preferably selected from the group comprising urine, blood, serum or blood plasma. However, the present invention shall not be restricted to this exemplary listing of types of samples.

In a further embodiment of the invention the subject suspected to suffer from a *Leptospira* infection could be a bird, amphibian, reptile or mammal, preferably wherein the mammal is a rodent or a human. Most preferred samples for the methods of the invention are samples which originate from humans.

LipL21 is detected in step b) of the present invention by the use of an antibody specific for the LipL21 protein. LipL21 is an outer membrane protein of pathogenic leptospires. It plays an important role in the infection process by acting as an adhesin, or as a target of specific antibodies, as porins, and as a receptor for soluble molecules and complement proteins.

In a preferred embodiment of the invention the detection in step b) in the above method is performed using a lateral flow test (LFT), for example using a competitive or non-competitive LFT.In a competitive LFT assay, the sample first encounters detectable particles which are labelled with an analogue of the analyte. In context of the present invention, the preferred analyte is the leptospiral cell, or fragments thereof, or more specifically, the LipL21 protein. The sample then encounters a test line containing antibodies specific to the analyte, e.g. LipL21.Thus the unlabelled analyte in the sample and the detectably labelled analogue compete for binding with the immobilized antibodies in the test line. The antibody used for a competitive LFT within the test line is usually a monoclonal antibody. In competitive LFTs the test line (or capture line) will be detectable in the event that the sample is negative for the analyte.

In non-competitive LFTs there is no binding-competition between unlabelled and labelled analyte. In this embodiment the analyte is first labelled with a detectable particle and then is brought into contact with an antibody specific for the analyte at the test line. Thus, in a non competitive LFT a detectable test line is indicative for the presence of the analyte in the sample.

In a specific embodiment the test or control lines in the assay of the invention can be detected by eye observation. In this regard, the detectable particle is preferably a colloidal gold particle.

In context of the present invention it is preferred that the LFT is a non-competitive LFT. Preferably the LFT comprises a test line and/or a control line for confirming the correct operation of the LFT. Such a control line contains antibodies directed to the detection antibody in the LFT and is located distal to the test line. At the control line any detectable particle coupled to an detection antibody is immobilized. In this way it is confirmed that the LFT contained undamaged detection particles. A detectable control line therefore confirms correct operation of the LFT.

In a second aspect the object of the present invention is solved by a diagnostic device for detecting the presence of *Leptospira* in a sample. The diagnostic device comprises:
a) a sample addition zone,
b) a conjugation zone, comprising an antibody-indicator complex composed of a polyclonal rabbit anti-recombinant LipL21 IgG antibody conjugated to an indicator,
c) a detection zone, comprising a zone of immobilized detection antibodies (capture line) and a control zone of immobilized control antibodies.

According to the present invention, the diagnostic device comprises a sample addition zone. As described herein this can be in the form of a pad (sample pad) that allows the introduction of the sample to be analysed onto the diagnostic device. Usually the sample is a liquid sample which is added onto the sample pad in order to start the assay. The sample pad preferably is made of a material that allows for a movement of the sample fluid by capillary forces and which therefore transports the analyte in the sample through the zones of the diagnostic device.

In a preferred embodiment, the sample pad material is a non woven cotton fibre, like cellulose fibre. It is further preferred that the sample pad for the purpose of the present invention permits the release of the analyte by high efficiency. To this end, the sample pad is pre-treated with salt buffer and a blocking agent, such as bovine serum albumin (BSA). In the preferred embodiment of the disclosure the pre-treatment is carried out using 20 mM phosphate buffer (pH 7.4) containing 0.1% (v/v) Tween 20, and 1% (w/v) BSA, and subsequent drying at 60°C. The sample pad is thereafter treated again with 20 mM phosphate buffer (pH 7.4) containing 2% (w/v) BSA, 0.1% (v/v) Tween 20, 3% (w/v) sucrose and 0.02% sodium azide and dried at 37°C.

The conjugation zone of the diagnostic device of the present invention has the function to allow the analyte to react with the antibody-indicator complex used in the present invention. The conjugation zone is dried before usage of the diagnostic device. The structure of the antibody-indicator complex with respect to the present invention is described herein below in more detail. When running the assay, the analyte within the sample liquid moves by capillary forces from the sample addition zone to the conjugation zone, wherein the analyte is exposed to the antibody-indicator complex. If analyte is present in the sample, the analyte may bind to the antibody-indicator complex. In any case the antibody-indicator complex is mobilized by the liquid in the sample and transported by capillary forces from the conjugation zone to the detection zone.

Preferred in the context of the present disclosure is, that the conjugation zone is in form of a pad (conjugation pad). A preferred material for the conjugation pad is glass fibre, e.g. MilliporeGlass FiberConjugatePadswith Lot Number- 92090602; 20 × 300 mm Millipore, MA, USA. Like the sample pad, the conjugation pad is preferably pre-treated with salt buffers and blocking agent. In a specific embodiment the pre-treatment is as follows: The anti-rLipL21 antibody labeled colloidal gold conjugate (antibody-indicator complex, was diluted (1:1 v/v) with 20 mM phosphate buffer (pH 7.4) containing 1% (w/v) BSA, 6% (w/v) sucrose. The conjugate pad pre-treated with 0.1% (v/v) Tween 20 for 24 hrs and dried for 45 minutes at 60°C was immersed into conjugate solution, then lyophilized and store in a desiccator at 4°C until use.

The detection zone of the diagnostic device according to the invention contains the test and control zones which allow to detect the absence or presence of the analyte. In one preferred instance the detection zone is made of a material that allows the liquid sample to move by capillary forces and on which the detection or control antibodies can be immobilized. Preferably is the use of a nitrocellulose membrane. For the purpose of the present invention, the zone of immobilized detection antibodies (capture line or test line) and, optionally, the control zone of immobilized control antibodies (control line) is arranged such that the sample liquid while the assay is run moves via capillary forces through the detection and/or control zone. The detection and/or control zone may be formed in any convenient way, such as sports, lines or likewise shaped zones.

Preferred is that the detection and/or control zone are formed as lines which are placed distant to each other to allow to distinguish their respective signals.

The absorbance zone is in a preferred embodiment composed of high-density cellulose material to allow the take up of excess sample fluid during the assay.

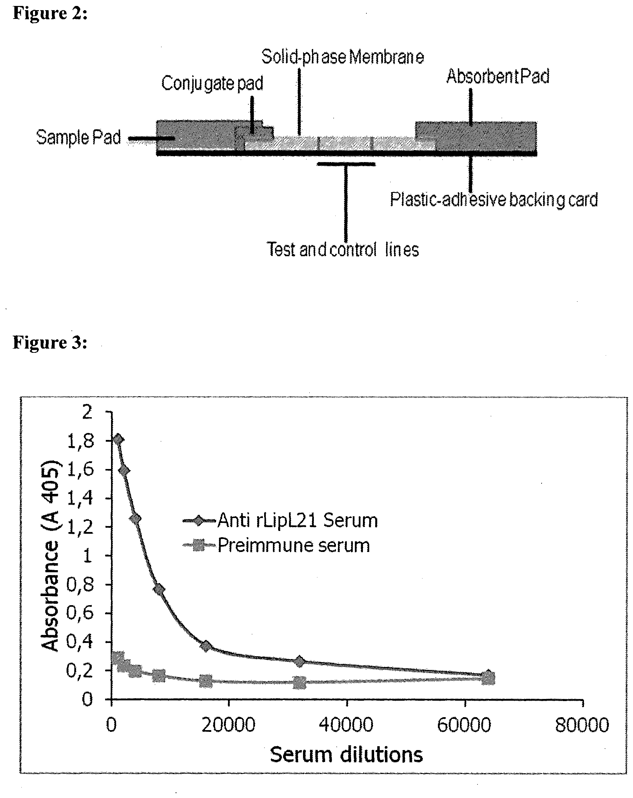

A preferred detection device according to the disclosure is exemplarily depicted in Figure 2.

For the purpose of the present invention zones a) to d) are in one embodiment arranged in successive sequence from the proximal to distal end of the device, preferably on a strip. The zones are preferably interconnected such that the sample fluid can move from zone a) to zone d) via capillary forces.

The diagnostic device comprises a polyclonal rabbit anti-LipL21 antibody, conjugated to an indicator, preferably wherein the indicator is a gold particle. Although gold particles are the preferred detectable labels in context of the present invention, any of a wide range of detectable markers/labels known in the art may be used.

Examples of detectable markers include, but are not limited to particles, luminescent labels; colorimetric labels, fluorescent labels; chemical labels; enzymes; radioactive labels; or radio frequency labels; metal colloids; and chemoluminescent labels.

Examples of common detection methodologies include, but are not limited to, optical methods, such as measuring light scattering, simple reflectance, luminometer or photomultiplier tube; radioactivity (measured with a Geiger counter, etc.); electrical conductivity or dielectric (capacitance); electrochemical detection of released electro-active agents, such as indium, bismuth, gallium or tellurium ions, or ferrocyanide.

The herein preferred use of colloidal gold particles allows for a visual inspection of the diagnostic device. In case the gold particles accumulate at the test line or control line, it shows as a red line. The signal is detectable by visual inspection.

In a next embodiment, the diagnostic device according to the invention comprises detection antibodies which are anti-LipL21 antibodies. As disclosed these can be polyclonal rabbit anti-LipL21 antibodies. Preferably the antibodies as described herein below.

The diagnostic device according to the invention further contains control antibodies which are anti-rabbit antibodies, preferably goat anti-rabbit antibodies.

The diagnostic device is preferred in one embodiment of the invention, wherein the line of immobilized detection antibodies (capture line or test line) contains the antibody in a concentration of between 500µg/ml to 10000µg/ml, as disclosed preferably 1000µg/ml, 1500µg/ml, 2000µg/ml, 2500µg/ml, 3000µg/ml, 3500µg/ml, 4000µg/ml, 4500µg/ml or 5000µg/ml, most preferably the concentration of the antibody is 2000µg/ml. Most preferred is that the line of immobilized detection antibodies (capture line or test line) contains 5µg of rabbit anti-LipL21 antibody and/or wherein the control line contains 1µg of goat anti-rabbit antibody.

For the purpose of the present invention the person of skill in the art is familiar with choosing different classes and origins of the detection and control antibodies. Antibodies raised in other species than rabbit, for example in goat, mouse or rat are also usable in the context of the present invention.

A further embodiment of the disclosure relates to the diagnostic device, wherein the distance between the capture zone and the control zone is about 5mm. The control zone is arranged such that it is placed on the device behind the test zone with respect to the capillary flow direction. The distance between both lines should be large enough to allow for an easy distinction between the test zone and the control zone. In this way the control zone allows to confirm the correct operation of the test system.

Yet another embodiment of the disclosure relates to the diagnostic device, wherein the diagnostic device can detect 10⁴ cells/ml of *Leptospira* in the sample to be analyzed. It is preferred that the diagnostic device of the present invention does not detect any one of the pathogens selected from the group comprising *Klebsiella pneumoniae*, *Pasteurella multocida*, *Proteus sp*, *Rhodococcus sp*, *Bacillussubtilis*, *Streptococcus mutans*, *Staphylococcus aureus*, *Salmonella typhi* or *Pseudomonas aeruginosa.* Most preferred is that the diagnostic device of the present invention detects specifically pathogenic *Leptospira* and not non-pathogenic *Leptospira,* such as saprophytic *Leptospira biflexa.*

In this way the present disclosure further provides methods and devices for the distinction of pathogenic *Leptospira* from non-pathogenic *Leptospira.*

In a further aspect of the present invention the object is solved by an *in vitro* method for diagnosing a *Leptospira* infection according to the appended claims. The method comprises the steps of providing a sample from a subject to be diagnosed, adding the sample to a diagnostic device according to the invention as described herein above, and wherein the visual appearance of the testzone is indicative for a *Leptospira* infection. Only in context of a non-competitive LFT assay, the appearance or positive detection of the test zone is indicative for the presence of the analyte and therefore indicative for the *Leptospira* infection. In case a competitive LFT assay is used in context of the invention, the above is similarly preferred, however, in such a test the appearance, or positive detection at the test zone, is indicative for the absence of the analyte and therefore is indicative for the absence of a *Leptospira* infection.

The test and/or control zones may be formed in any convenient way, such as sports, lines or likewise shaped zones. Further preferred is that instead of only one test and/or control zone a multitude of test and/or control zones are used in order to further specify the assay result. In the context of the present invention the terms "capture zone" or "test zone" are used interchangeably.

The in vitro method of diagnosis according to the invention is in a preferred embodiment a method, wherein the sample is a biopsy or a liquid sample. As disclosed this can be a kidney biopsy, or a liquid sample, preferably selected from the group consisting of urine, blood or serum. In general any body fluid or tissue that is, or can be, infected by the *Leptospira* cells in the subject suspected to suffer from such an infection can be the source of the samples usable in the methods and/or devices of the present invention.

The subject suspected to suffer from a *Leptospira* infection in one preferred embodiment is a bird, amphibian, reptile or mammal, preferably wherein the mammal is a rodent or a human. Due to the surprising effectiveness of the methods and devices described herein, the method according to the invention is a method, wherein the subject to be diagnosed is in the early stage of *Leptospira* infection, within Day 1 to Day 7 of *Leptospira* infection. As disclosed preferably within Day 1 to Day 5 of *Leptospira* infection, more preferred the subject to be diagnosed is in Day 1, 2 , 3, 4, 5, 6, 7, 8, 9, 10 after the infection, or in a later stage.

In a preferred embodiment of the method according to the invention, the sample is added to the sample addition zone of the diagnostic device.

In another embodiment, a method is preferred wherein the appearance of a control zone is indicative for the correct operation of the assay. In one embodiment it is preferred that the diagnostic device is a competitive lateral flow test (LFT). In a competitive lateral flow test it is preferred that the appearance of a test zone indicates the absence of a *Leptospira* infection.

In a third aspect the object of the present disclosure is solved by an antibody which is specific for *Leptospira*, preferably wherein the antibody binds specifically to the membrane protein LipL21.In one embodiment the antibody of the disclosure is a polyclonal rabbit anti-LipL21 antibody or a polyclonal rabbit anti-r (recombinant) LipL21 antibody. A polyclonal antibody provides that the antigen is detected via multiple epitopes.

In a fifth aspect the object of the present invention is solved by the use of a diagnostic device according to the appended claims.

It is preferred in the context of the above described embodiments of the present invention that the LFT assay is run for 20 minutes starting from sample addition until the signals of the capture and/or control zones are analysed.

While the present invention has been described with specificity in accordance with certain of its preferred embodiments, the following examples serve only to illustrate the invention and are not intended to limit the invention.

### Description of the Drawings

- **Figure 1:**: Schematic flow chart of the antibody production
- **Figure 2:**: Schematic representation of the lateral-flow immunoassay strip
- **Figure 3:**: Shows the representation of the purified rLipL21 (50ng) reacted by ELISA to anti-rLipL21 serum with rabbit pre immune serum.
- **Figure 4:**: Anti-rLipL21 IgG was purified from rabbit antiserum against rLipL21. Lane L, load anti-rLipL21 serum; Lane FT, Flow thru of unbound proteins from column; Lane W, Wash of non specific proteins from column; Lane 1, eluted fraction without protein, Lane 2, purified anti-rLipL21 IgG and Lane 2, Concentrated anti-rLipL21 IgG.
- **Figure 5:**: Localization of LipL21 from *Leptospira spp* (Patoc 1 - L. biflexa, Pomona and RGA - L. interrogans) proteins during membrane fractionation by Triton X-114 method. Western blot of SDS-PAGE- used Triton X-114 fractions with anti-rLipL21 IgG (lane D. Detergent phase; lane S, soluble phase). Positions of standard molecular mass markers are indicated on the left.

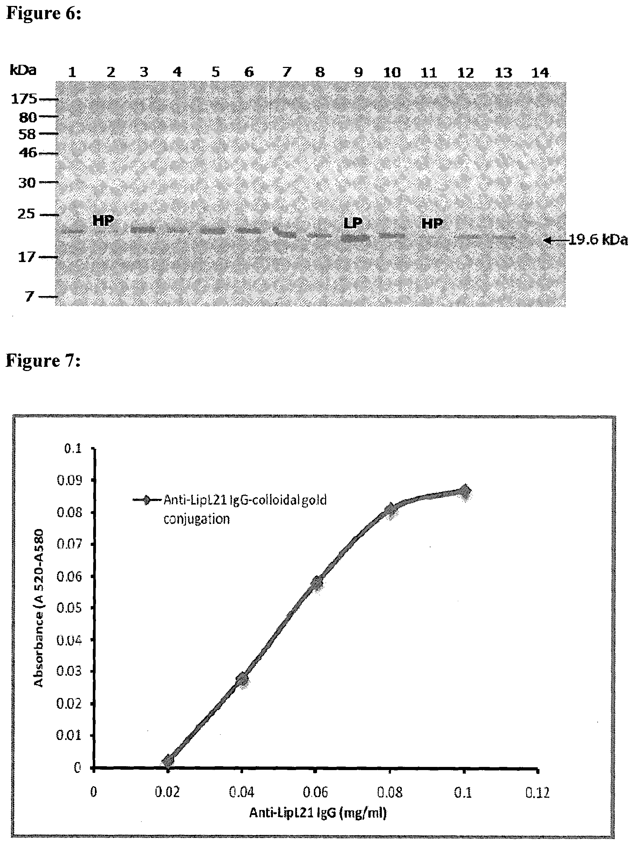

- **Figure 6:**: Immunoblot of a panel of *Leptospira spp* obtained by using rabbit rLipL21 IgG to detect single band in leptospiral LipL21 antigen in detergent phase. Lane 1, Canicola (strain Hond Utrecht IV); Lane 2, Hardjobovis (Sponselee); Lane 3, Autumnalis (strain Akiyami A); Lane 4, Icterohaemorrhagiae (strain RGA); Lane 5, Pyrogenes (strain Salinum); Lane 6, Pomona (strain Pomona); Lane 7, Grippotyphosa (strain Moskva V); Lane 8, Leptospira kmetyi serovar Malaysia strain Bejo-iso9 ; Lane9, R44 (local isolate); Lane 10, Grippotyphosa (strain Moskva V); Lane 11, Hebdomadis (strain Hebdomadis); Lane 12, Australis (strain Ballico); Lane 13, Balum (strain Mus 127); Lane 14, non-pathogenic species L. biflexa (strain Patoc 1).
- **Figure 7:**: Optimization of conjugation between anti-rLipL21 antibody and colloidal gold.
- **Figure 8:**: A representative photograph of a positive (*Leptospira* culture) and negative (blank EMJH) samples. Anti-rLipL21 antiserum is the capture antibody used to detect leptospiral LipL21 (positive) from infected host during leptospiremia in acute leptospirosis.
- **Figure 9:**: LFT strips analyses with different concentration (0.2, 0.5, 1, 2, 5, 10 µg) of rLipL21, Upper line is the control line, bottom line test and control tested with only diluted buffer.
- **Figure 10:**: LFT strips analyses with different serovars of pathogenic *Leptospira* spp and non pathogenic strain L. biflexa strain Patoc 1.
- **Figure 11:**: Sensitivity, specificity and accuracy values (PPV and NPV) of the developed LFT strips to detect pathogenic leptospires in infected hamsters as a reference standard.
- **Figure 12:**: Sensitivity, specificity and accuracy values (PPV and NPV) of the *Leptospira* isolation to detect pathogenic leptospires in infected hamsters as a reference standard.
- **Figure 13:**: Sensitivity, specificity and accuracy values (PPV and NPV) of the PCR to detect pathogenic leptospiral *lipL21* gene using infected hamsters as a reference standard.

### Examples

### Example 1: Anti-LipL21 polyclonal antibodies produced from rabbit

### Immunization

Antiserum to LipL21 was prepared by immunizing a rabbit with the purified rLipL21 protein as described previously (Shang et al., 1996). In this study, two rabbits were immunized using 100 µg of purified rLipL21. Purified rLipL21 protein was loaded onto SDS-12% polyacrylamide gel and allowed to migrate into the separating gel during electrophoresis. An rLipL21 containing band was excised from the gel and desiccated. Desiccated gel containing recombinant protein were ground to a powder, dissolved in 1ml of water and mixed with 1ml of complete Freund's adjuvant (Merck, USA).(Merck). Pre-immune serum was collected before immunization and used as a control in neutralization experiments. The mixture of rLipL21 and complete adjuvant were injected both subcutaneously and intramuscularly on Day 1 into leptospiral antibodies free New Zealand White rabbits. Additional immunizations with the same dosage of the rLipL21 with incomplete Freund's adjuvant (Merck) were done on Day 15 and Day 29. On Day 36 the rabbits were bled by heart puncture and the serum was collected to detect antibodies against LipL21. The LipL21-antiserum was stored in small aliquots at -20°C until use. Animals were housed in accordance with the ethical principles and experimental procedures with animals were approved by Animal Care and Use Committee of the Faculty of Veterinary Medicine, University Putra Malaysia (AUP No: 09R57/Mac 09-Feb10).

### Purificationof IgG from rabbit polyclonal antibody serum

These methods effectively purify the main classes of antibodies (IgG, IgM, and IgA). A protocol for affinity chromatography purification of polyclonal antibodies with immunoglobulin class is also provided. However the ion-exchange chromatography is indicated for purifying intact monoclonal and polyclonal antibodies and antibody fragments from different sources. IgG purification from whole serum by using ammonium sulfate precipitation effectively removes other protein contaminations, but they still contain both the specific (to the desired antigen) and non-specific IgG molecules. These nonspecific contaminations can be removed by size-exclusion or ion exchange chromatography. Instead, Protein A, protein G or one of the newer commercial proprietary affinity chromatography columns can be used (Grodzki and Berenstein, 2010).

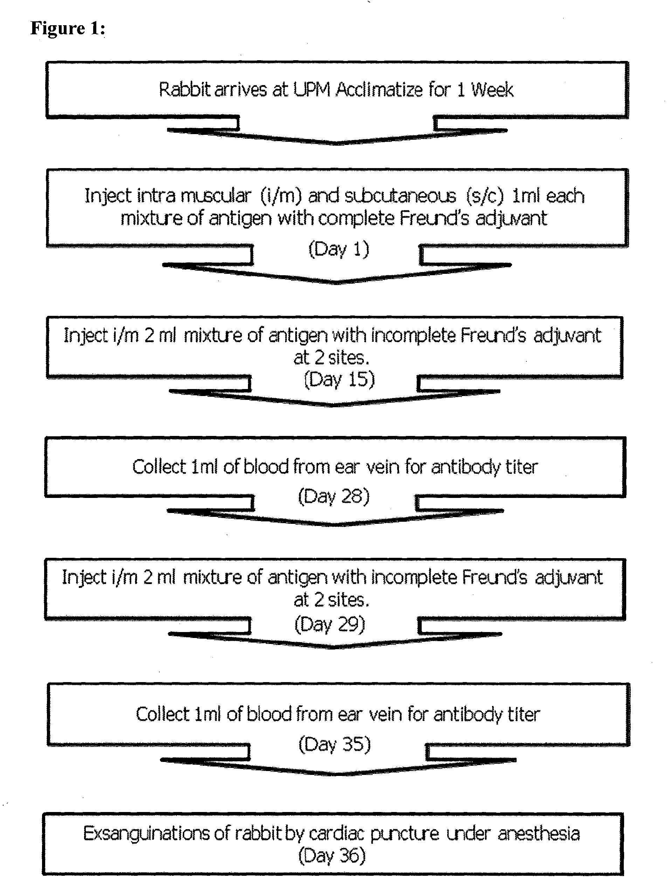

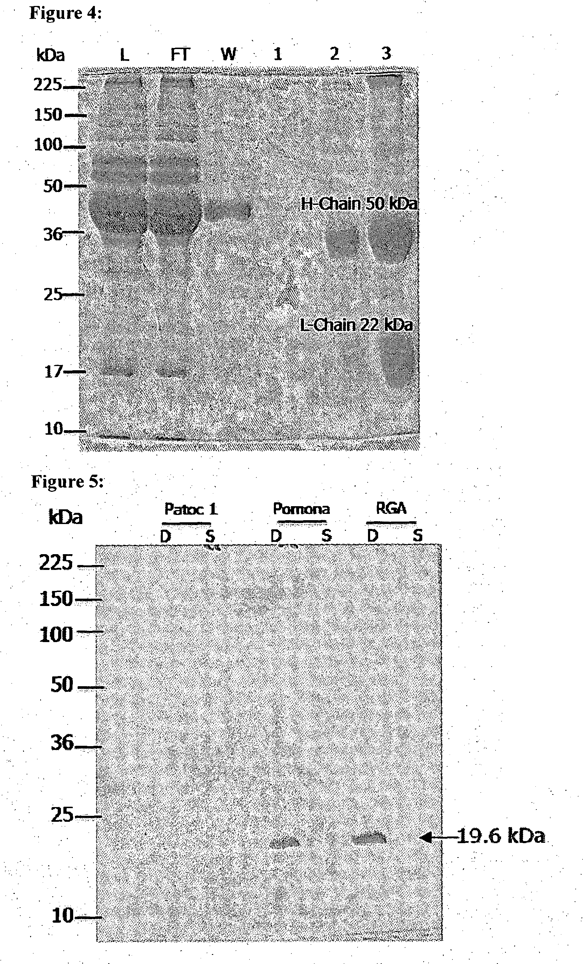

To obtain polyclonal purified immunoglobulin, whole serum supernatant was used in Montage Antibody Purification Kits with PROSEP-A (LSK2 ABA 20, Millipore). A schematic representation of the anti-body generation is found in Figure 1. Immunized rabbit antiserum containing, IgG were purified according to the manufacturer's instructions to Brief, The PROSEP-A media was Pre equilibrated with 10 ml Binding Buffer A by centrifuged the spin column at 500 x g for 5 minutes. The serum sample to be loaded to the spin column was prefiltered through a 0.22 µm Steriflip-GP device to remove any debris immediately before loading the sample. The filtered serum sample was diluted with 1:1 v/v in Binding Buffer A. (For example, 10 ml filtered sample was added to 10 ml binding buffer) 20 ml of the sample was allowed to flow through the spin column. The column was spun at 150 × g for 20 minutes, unbound contaminants were removed by adding 10 ml Binding Buffer A and the spin column was spun for 2 minutes at 500 × g. Above wash procedure was carried out once again. The unbound wash contained non-immunoglobulin components. Finally the rabbit IgG was eluted by adding 10 ml Elution Buffer B2 directly into a fresh centrifuge tube containing 1.3 ml Neutralization Buffer C to bring the sample to neutral pH, by spinning the column for 5 minutes at 500 × g. The purified IgG was analyzed for the quality by SDS-PAGE followed by CBB staining. The total immunoglobulin was quantified spectrophotometrically, and ELISA was performed to confirmed reactivity of the IgG towards rLipL21 (Figure 3). Two micrograms of purified IgG proteins was analyzed for reactivity against rLipL21 and leptospiral OMPs by Western blotting (Figure 4). The purified IgG proteins were stored at -70°C for further analysis.

### Localization of LipL21 via detection of anti-rLipL21IgG:

For the investigation of LipL21-localization in *Leptospira* spp, the leptospiral outer membrane was extracted by the nonionic detergent Triton X-114 phase separation. LipL21 protein was observed in the detergent phase, but not observed in soluble phase (Figure 5). When 20mM CaCl₂ was added, LipL21 was exclusively in the detergent-phase. LipL21 was found to partition exclusively into the detergent phase. This is in contrast to the results of the immunoblot, where the anti-rLipL21 IgG reacted specifically to the protein having a size corresponding with the molecular mass predicted for LipL21 on detergent phase (Figure 5). The detergent phase of leptospiral OMP interaction with anti-rLipL21 IgG indeed originated from interactions with leptospiral LipL21. No reactivity was evident at the membrane fractions from non-pathogenic strains.

### Specificity of anti-rLipL21 IgG against leptospiral LipL21 in immunoblot:

The specificity of antibodies purified from antisera generated in rabbits was first evaluated by immunoblotting against the rLipL21 fusion protein. The purified anti-rLipL21 IgG showed high specificity to their corresponding recombinant antigens. The specificity of the antibodies against each individual leptospiral serovar protein was further evaluated by immunoblotting analyses of Triton X 114 fractionation-separated OMP (Figure 5). Similarly, antiserum raised against rLipL21 in the rabbit was tested against leptospiral OMP fractions obtained by Triton X 114 method. More importantly, LipL21 from OMP detergent fractions were detected in the tested *Leptospira* strains by immunoblot, which could be observed at approximately predicted molecular weight 19.6 kDa (Figure 6), and similarly the lipL21 gene of these strains could be detected by PCR amplification. Pre immune serum (prior to rabbit immunization with purified, recombinant protein) was tested and found not to be reactive with any leptospiral antigens. In immunoblotting analyses, the anti-rLipL21 detects LipL21 from pathogenic *Leptospira* spp without cross reactivity of particular importance was the fact that anti-rLipL21 IgG did not cross-react with the other bacterial antigens tested. This specificity reduces the possibility of a false positive result in diagnostic kit of the present disclosure. Although the anti-rLipL21 IgG was detected from each of the LipL21 pathogenic *Leptospira* spp, the intensity of band differed between culture passages. Weak cross-reactions were observed with 3 strains of related serovars (Canicola, Pyrogenes and Grippotyphosa), demonstrating the specificity of the LipL21 antiserum (Figure 6). LipL21 has strong sequence conservation as is observed in the lipL21 genes of *Leptospira* spp. The OMP separation used relatively the same amount of leptospiral pathogens for immunoblot. However, the present results of immunoblot showed the anti-rLipL21 IgG, detected a very strong band on the low passage (LP) of *Leptospira* spp and low intensity band with the high passage (HP) of *Leptospira* spp.

However, antibodies against rLipL21 proteins clearly detected leptospiral antigens with anti-rLipL21 being the most sensitive. The anti-rLipL21 IgG reacted very similarly with all pathogenic leptospiral OMP containing detergent fractions, but selectively detecting LipL21 indicating that they may have affinity to the same leptospiral epitope. The polyclonal antibodies can be highly sensitive but less specific then monoclonal antibodies while monoclonal antibodies can be highly specific with less sensitivity (Varma*et al*., 2002). Usage of highly sensitive antibody like anti-rLipL21 IgG will give potentially good results in diagnostic kits.

### Example 2: Development of Lateral Flow Test strips

### Optimization of conjugation between anti-rLipL21 IgG antibody and colloidal gold

Colloidal gold particles were conjugated with polyclonal antibodies and used as detector reagents. The main advantage of using colloidal gold particle is it provides the opportunity to improve LFT strips sensitivity and is less susceptible to aggregation during the preparation of the LFT strips (Chiao *et al*., 2004). In this rapid test, the colloidal gold particle (40nm) was conjugated with anti-rLipL21 IgG. The conjugated antibody was directly absorbed on colloidal gold particle surface, mediated by London-Van der Waals force and hydrophobic interaction (Hermanson *et al*., 1992). Optimal concentration of antibody (0.08-0.1 mg/ml) for the conjugation with colloidal gold was determined by comparing the absorption between 520-580 nm (Figure 7).

### Conjugation of anti-rLipL21 IgG -colloidal gold complexes

For conjugation, each purified anti-rLipL21 IgG solution was used in 10% excess of the minimal amount. 100 ml of colloidal gold (pH 9.0) was swiftly added to 10 ml of the abovementioned purified anti-rLipL21 IgG solution with gentle stirring. Then the solution was incubated for 1 hour, and then non specific binding area was blocked with BSA (final concentration 1%) and PEG (final concentration 0.01%) for mixed for 30 minutes to incubate another 1 hour. The mixture was centrifuged (15,000 rpm) at 4°C for 45 minutes, and the colorless supernatant was carefully aspirated and discarded. Subsequently, the loose sediment of gold-labelled antibody was resuspended with 20 ml of 20 mM PBS, pH adjusted to 9.0 containing 1% BSA and 0.01% PEG, followed by another centrifugation. This procedure was repeated twice to ensure clean up of the free (unlabelled) purified anti-rLipL21 IgG. Finally, the liquid droplet of purified anti-rLipL21 IgG- gold complex was dissolved in 10 ml of 20 mM PBS (pH adjusted to 9.0) involving 10% sucrose, 1% BSA, and 0.01% sodium azide. The gold labelled purified anti-rLipL21 IgG was stored at 4°C for the next use.

### Preparation of the sample pad

The Cellulose Fibre sample pad is a non woven cotton fibre is used as a sample and absorbent pad (Millipore Cellulose Fiber Sample Pads with Lot Number- 92090602; 20 × 300 mm Millipore, MA, USA) in LFT kit. The properly treated sample pad is compatible with the assay, and releases the analyte very efficiently. The sample pad pre-treatment was carried out using 20 mM phosphate buffer (pH 7.4) containing 0.1% (v/v) Tween 20, and 1% (w/v) BSA was dried at 60°C and kept in desiccators until use. Sample pad was again treated with 20 mM phosphate buffer (pH 7.4) containing 2% (w/v) BSA, 0.1% (v/v) Tween 20, 3% (w/v) sucrose and 0.02% sodium azide and dried at 37°C.

### Preparation of the conjugated pad

The role of the conjugate pad (Millipore Glass Fiber Conjugate Pads with Lot Number-92090602; 20 × 300 mm Millipore, MA, USA) in LFT is to allow the conjugate, hold it stable over its entire shelf life, and release it efficiently and reproducibly when the assay is run. The colloidal gold conjugated anti-rLipL21 antibody containing conjugate pad was prepared for immobilization. The anti-rLipL21 antibody labeled colloidal gold conjugate was diluted (1:1 v/v) with 20 mM phosphate buffer (pH 7.4) containing 1% (w/v) BSA, 6% (w/v) sucrose. The conjugate pad pre-treated with 0.1% (v/v) Tween 20 for 24 hrs and dried for 45 minutes at 600C was immersed into conjugate solution, then lyophilized and store in a desiccator at 40C until use.

### Preparation of the absorbent pad

The high-density cellulose material (Millipore Cellulose Fiber absorbent Pads with Lot Number-92090602; 20 × 300 mm Millipore, MA, USA) is used as an absorbent pad. This is designed to absorb the excessive fluid added to the LFT.

### Preparation of lateral-flow immunoassay strip

The composition of the lateral-flow immunoassay device is shown in Figure 2. The test device was prepared as follows: The control line of goat anti-rabbit IgG (1 µg) and test line of rabbit anti-rLipL21 IgG (5 µg) (in 50 mM sodium phosphate-buffered saline, pH 7.4) were separately applied near to one end of nitrocellulose membrane (High-Flow plus 180 membrane card 60 mmx301 mm Millipore, MA, USA) with the help of a micro pipette. The distance between the test line and control line was about 5mm. The test strips were dried at 37°C for 30 minutes. Then nitrocellulose membrane was blocked by immersing the strips in PBS containing 1% BSA for 30 minutes at RT. The test strips were washed and dried, and then stored in a desiccator at 4°C.

The pre-treated sample pad, colloidal gold antibody saturated conjugate pad, nitrocellulose membrane (containing anti-rLipL21 antibody for test line and goat anti-rabbit IgG for control line) and absorption pad were assembled and attached to a plastic backing. The conjugate pad was attached to the top of the membrane with 1-2 mm overlapping on the membrane, and then the sample pad was attached to the top of the conjugate pad in a similar manner. The absorbent pad was attached to the bottom of the membrane with 1-2 mm overlapping on the membrane also. The LFT kit were then enclosed in the plastic box and sealed in the aluminum foil bag containing desiccant gel. The LFT kit was ready for use in the assay and stored under dry conditions at room temperature until use.

### Example 3: Use of the LFT Strip

The LFT device detection zone containing the rabbit anti-LipL21 IgG and goat anti-rabbit IgG are separately stripped onto test line and control line. The purified anti-LipL21 IgG coated colloidal gold particles react with goat anti-rabbit antibody and can be immobilized in control region, while the LipL21 or leptospires bound purified anti-LipL21 IgG-coated colloidal gold particles react with anti-LipL21 antibody and can be immobilized in test region to form a mobile sandwich complex. The sample was absorbed by the sample pad. Due to capillary forces this mixture moved towards the absorbent pad. In doing so, all remaining active sites of the cellulose nitrate membrane were blocked by BSA and/or Tween-20. If detectable LipL21 was present, a positive test result was achieved, which was visible by the appearance of a coloured test line and control line. The negative result is determined by the appearance of only single line in control region. The visible colour intensity of the test line correlated with the antigen concentration present in the sample. Excess sample that flows beyond the test and control line was taken up by the absorbent pad.

A portion of blood/serum sample was transferred directly onto the sample pad. The LFT was incubated laterally for 10-15 minutes, and the sample solutions absorbed and immediately dissolved the colloidal gold conjugate, the reaction between the conjugate antibody and LipL21 antigen took place, and this complex was carried forward onto the nitrocellulose membrane containing the capture lines (Test line containing anti-LipL21 IgG and control line containing anti-rabbit IgG). The nitrocellulose membrane containing the second antibody interaction with leptospiral LipL21-conjugated antibody complex would take place. This immune complex observed on the nitrocellulose membrane solid surface and the unbound reagent was subsequently adsorbed by the absorbent pad. When two dark red lines were observed after incubation at test line and the control line, sample evaluation was defined as positive. In the case of observation of only one dark red line at the control line, sample evaluation was defined as negative.

### Example 4: Optimization of the LFT Strip

### Temperature:

The effects of various temperatures on the naked eye values were evaluated. In comparison with 4°C, the values at 26°C, and 37°C were slightly changed, however, with no significant difference. No obvious changes could be observed at various examined temperatures compared to the test's performance at 4°C. The results indicated that the determination of results of test assay with the naked eye may be affected at the higher temperature.

### Reading Time:

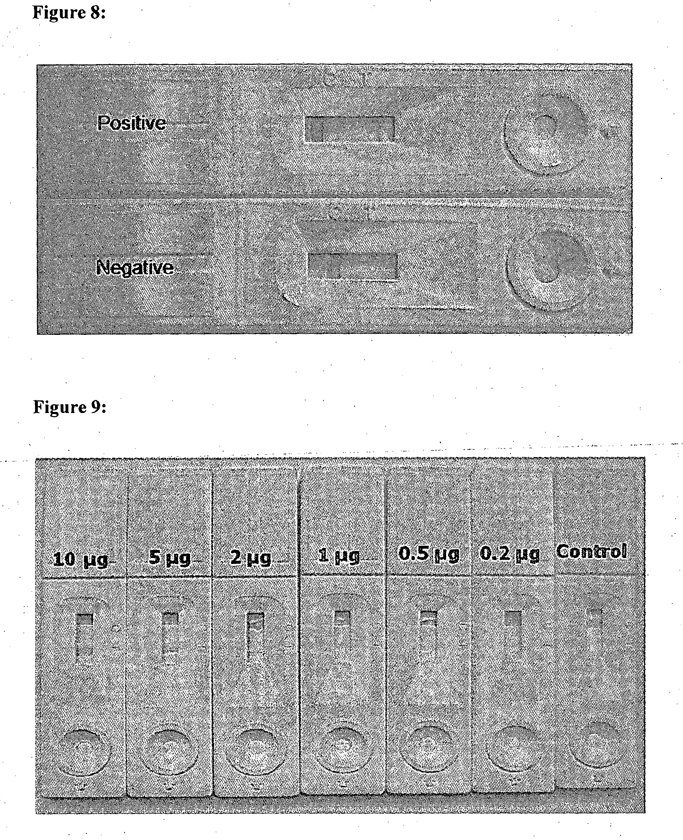

The time for appearance of a dark red color on the test lines was optimized for both positive control and for samples with low concentrations of antigens (Figure 8). The results were observed after 15-25 minutes. The colloidal gold-labelled antibodies bind to the leptospiral OMP of LipL21 in the capture line (anti-rLipL21 IgG) on the nitrocellulose membrane. If the leptospires levels in the blood samples are negative or below the particular level, the time for the LFT strip to develop a color was 20 minutes. The difference between positive and negative samples could easily be distinguished with the naked eyes. Thus, the LFT strip assay time was standardized to 20 min.

### Sensitivity:

In the present study, the leptospires number and concentration of rLipL21 and capture antibody (anti-rLipL21 IgG) were optimized (Figure 9). The capture line contains anti-rLipL21 IgG in different concentrations, such as 500 µg/ml, 1000 µg/ml, 1500 µg/ml, 3000 µg/ml, 4000 µg/ml, and 5000 µg/ml of anti-rLipL21 IgG (Table 1). The colloidal gold based LFT strips should provide a rapid visual qualitative test which gave a simple reactive or non reactive response to the levels of the target antigens. The cut-off value with the naked eye was defined here as the amount of leptospires and capture antibodies (anti-rLipL21 IgG) in the standard samples that resulted in dark red colour development at the test lines. In accordance with visual evaluation, the cut-off value of capture antibodies (anti-rLipL21 IgG) showed 2000µg/ml (Table 1).

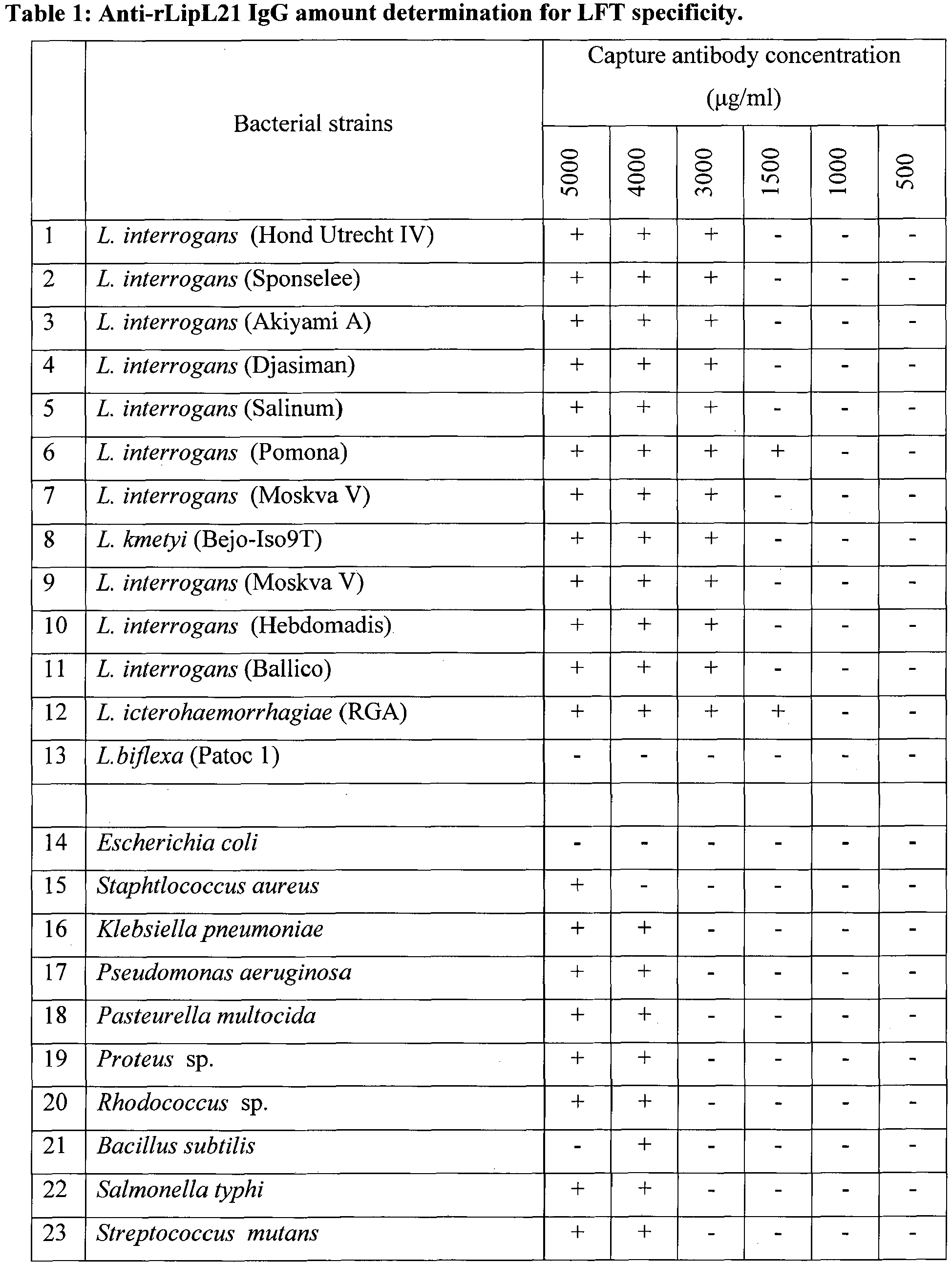

The preparation of leptospiral cells comprised centrifugation of cell culture suspensions (>10⁷ cells/ml) at 8000 × g for 10 min at 4°C, which was followed by dilution in PBS. Then 200 µl of 10⁶, 10⁵, 10⁴, 10³, 10² and 10¹ dilutions of leptospires were used to determine the copy number of the leptospires in LFT strips (Table 2). In the LFT analyses, the samples containing copy number of leptospires was 10⁴ cells/ml or more displayed positive, similar results were observed by repeating the test using different samples 5 times, indicating the high reproducibility of this experimental system.

### Specificity:

The LFT strips were based on the non-competitive principle, the direct relationship between concentrations of antigens and antibodies in sample and development of dark red colour on the test lines. Therefore, the detection limits of the test strips should be determined by testing the leptospires number in the standard laboratory cultures, minimal concentration of rLipL21 (50µg, 20µg, 10µg, 5µg, 2µg, 1µg) and anti-rLipL21 IgG in the LFT strips (Figure 9). The intensities of the bands for various concentrations of leptospires on the test lines gradually declined from the strongest band observed at above 10µg rLipL21.

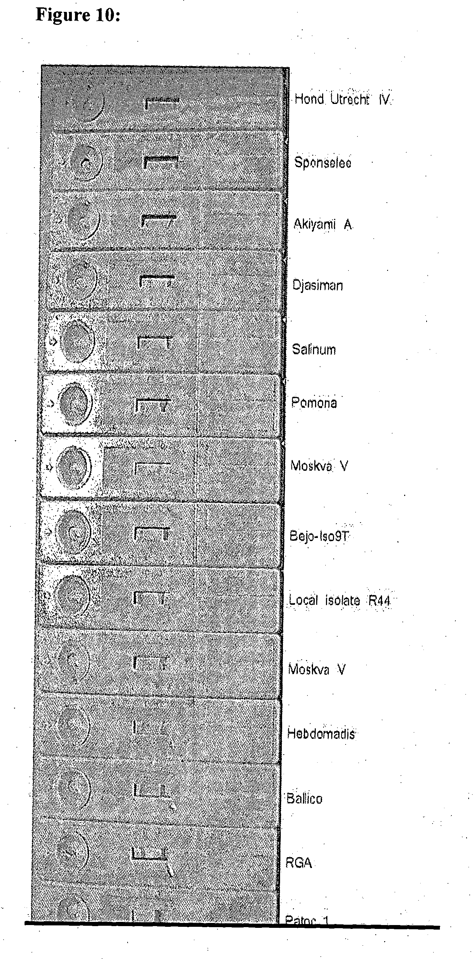

To determine the specificity of the LFT strips with standard antigens of *Leptospira* spp and other bacteria, they were characterized using the LFT strips simultaneously. Clearly, among all of the tested pathogens, leptospires showed strong band on the test and control line, which was not observed on non-pathogenic leptospires (Figure 10). This suggests that the LFT strips can specifically detect pathogenic leptospira but not others tested, such as *K. pneumoniae*, *P. multocida, Proteus sp, Rhodococcus sp, B. subtilis, S. mutans, S. aureus, S. typhi, P. aeruginosa,* and non-pathogenic *L. biflexa* (Table 1 and 2).

**Table 2: Antigen amount determination for LFT sensitivity.**

| | Bacterial strains | Number of cells/ml | | | | | |
|---|---|---|---|---|---|---|---|
| | | 10⁶ | 10⁵ | 10⁴ | 10³ | 10² | 10¹ |
| 1 | *L. interrogans* (Hond Utrecht IV) | + | + | + | - | - | - |
| 2 | *L. interrogans* (Sponselee) | + | + | + | - | - | - |
| 3 | *L. interrogans* (Akiyami A) | + | + | - | - | - | - |
| 4 | *L. interrogans* (Djasiman) | + | - | + | - | - | - |
| 5 | *L. interrogans* (Salinum) | + | + | + | - | - | - |
| 6 | *L. interrogans* (Pomona) | + | + | + | - | - | - |
| 7 | *L. interrogans* (Moskva V) | + | + | - | - | - | - |
| 8 | *L. kmetyi* (Bejo-Iso9T) | + | + | + | - | - | - |
| 9 | *L. interrogans* (Moskva V) | + | + | + | - | - | - |
| 10 | *L. interrogans* (Hebdomadis) | + | - | + | - | - | - |
| 11 | *L. interrogans* (Ballico) | + | + | + | - | - | - |
| 12 | *L. icterohaemorrhagiae* (RGA) | + | + | - | - | - | - |
| 13 | *L.biflexa* (Patoc 1) | - | - | - | - | - | - |
| | | | | | | | |
| 14 | *Escherichia coli* | - | - | - | - | - | - |
| 15 | *Staphylococcus aureus* | - | - | - | - | - | - |
| 16 | *Klebsiella pneumoniae* | - | - | - | - | - | - |
| 17 | *Pseudomonas aeruginosa* | - | - | - | - | - | - |
| 18 | *Pasteurella multocida* | - | - | - | - | - | - |
| 19 | *Proteus* spp | - | - | - | - | - | - |
| 20 | *Rhodococcus* spp | - | - | - | - | - | - |
| 21 | *Bacillus subtilis* | - | - | - | - | - | - |
| 22 | *Salmonella typhi* | - | - | - | - | - | - |
| 23 | *Streptococcus mutans* | - | - | - | - | - | - |

### Example 5: Experimental Verification

### Evaluation of the LFT strips for testing samples from experimental infected hamsters

The LFT strips were evaluated with blood culture and PCR was performed in the infected hamster's experimental model (Table 3). This animal model condition is as close as possible to those in a natural infection and is infected with serovar Icterohaemorrhagiae, which was isolated from field rat from Malaysia. These kinds of rodents could be one of the animals responsible for the leptospirosis as reported by Vedhagiri *et al*., (2010) in India. The *in vivo* efficiency of LFT strips sensitivity revealed that they could detect rLipL21 protein as low as 2 µg within 15 minutes, which was similar to that of *Leptospira* isolation in infected animal model. Fifteen leptospirosis infected animal models and five uninfected animals as control were tested for the presence of the corresponding leptospires by Leptospira isolation and PCR using lipL21 gene specific primers. The absence of infection was also confirmed by *Leptospira* isolation. All the LFT strip-positive samples were positive by *Leptospira* isolation on Day 1 to Day 5 post infections, when no clinical sign was observed in all experimental hamsters (Table 3). During these animal experiments, there were no false positive results with all three methods. With the LFT strips, all infected blood samples were tested positive with the exception of false negative results. Following the hamster infection, their blood samples were longitudinally collected and tested using the LFT strips described in Table 4. Control hamsters did not show positivity for leptospirosis infection throughout the observation period. There were no false positive LFT strips results with control animals.

The overall sensitivity and specificity of each test results are shown in Table 4. Although the sensitivity of the LFT strips was 73.3% (55/77), it had 100% specificity with infected animal blood samples. Similarly the isolation of leptospires from infected animal model shows 100% specificity with 74.7% (56/75) sensitivity. The LFT strips and the Leptospira isolation both had high sensitivities and specificities. The PCR was the least sensitive of the assays, and the results may be varied by using different primers. Because LipL21 gene specific *Leptospira* isolation is considered the "gold standard," all of the infections identified by antigen detection were confirmed to be *Leptospira* spp by this method.

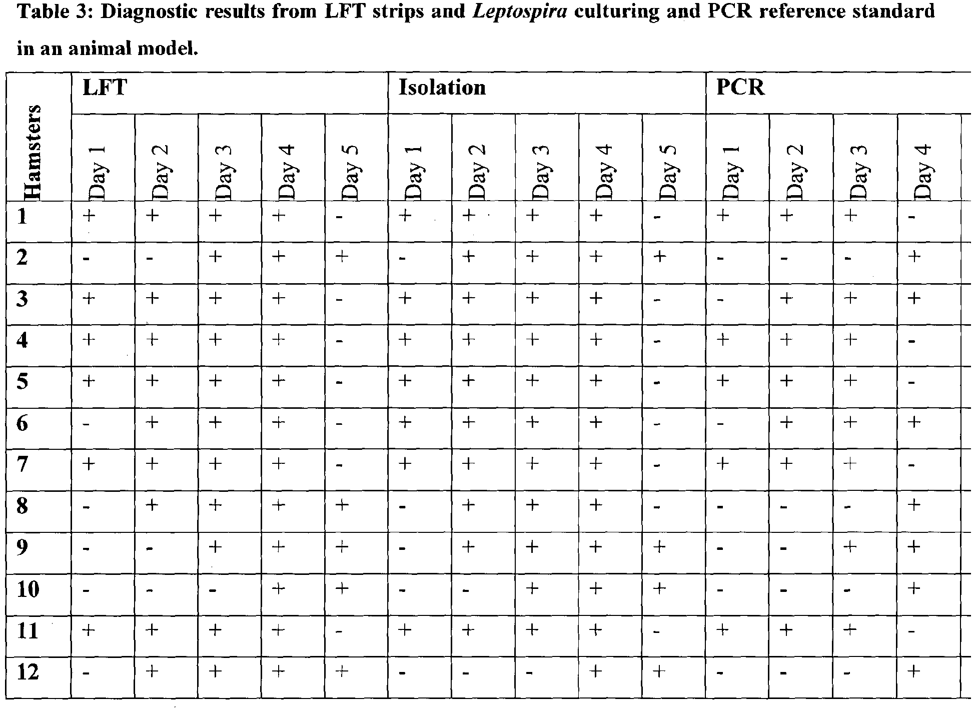

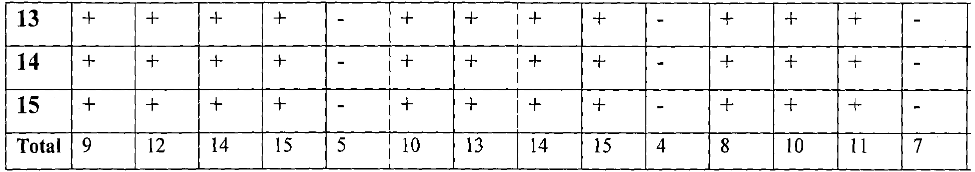

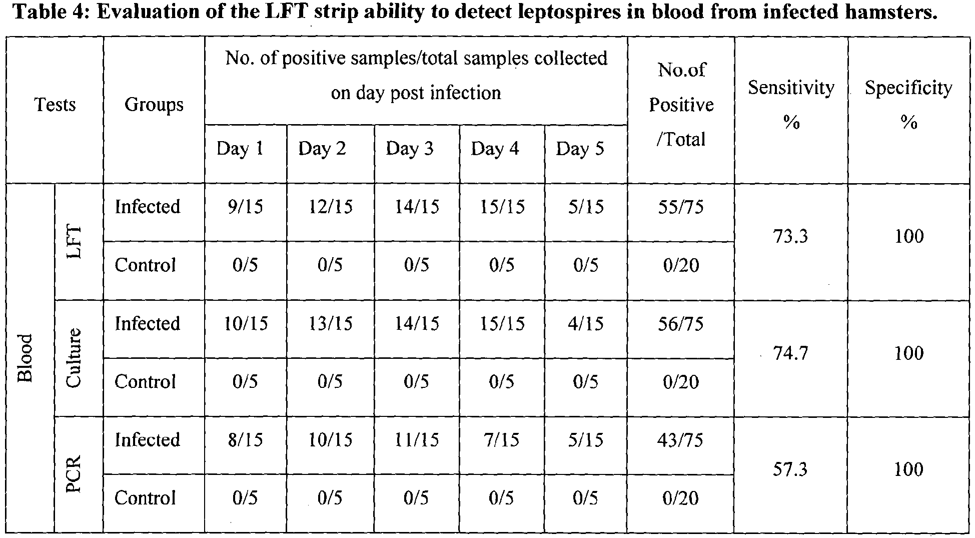

### Detection of leptospires in blood using LFT strips

Although leptospirosis is an endemic disease in many countries including Malaysia, cases of leptospirosis often remain underestimated and were treated as fever of unknown causes (El Jalii, *et al*., 2002). Culture isolation still represents the gold standard of laboratory diagnosis. The sensitivity of culture method could be enhanced by using blood or urine sample from *Leptospira* infected cases, but making media and maintaining culture still remains an invasive, tedious and time consuming technique, therefore diagnostic kit is generally preferred for acute leptospirosis.

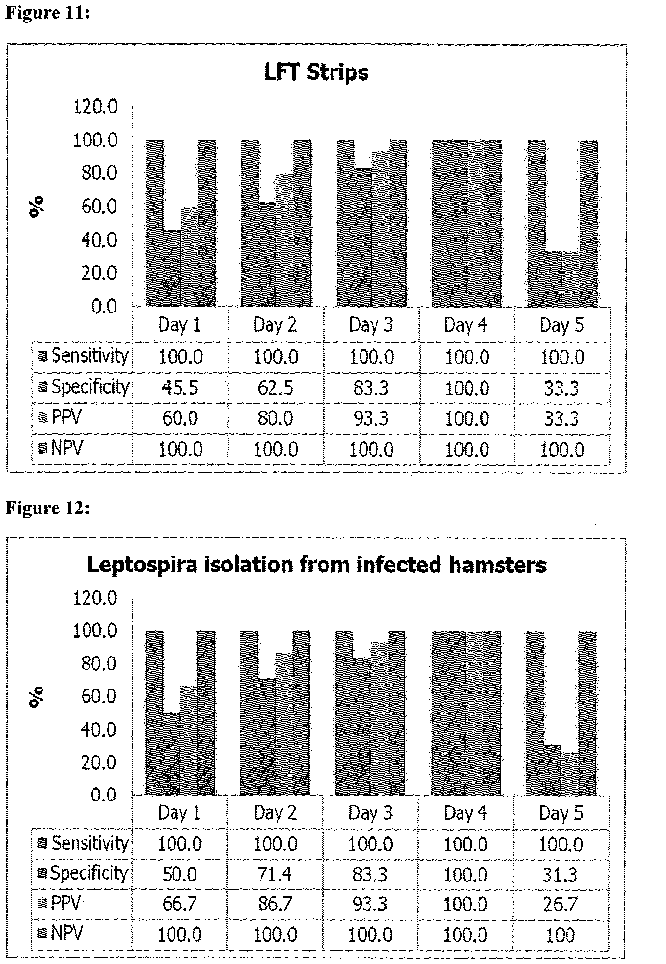

The LFT strips could effectively detect leptospirosis by Day1 to Day 4 post infection in hamster model (Table 5 and Figure 11). The early detection was possible because leptospires grows faster and circulates rapidly in infected animal's blood (Truccolo *et al*., 2002). The LFT strips specificities and negative predictive values were observed at 100% in the infected animal models. The LFT strips have thus proven to be a useful method for diagnosis of acute leptospirosis. Leptospiral infection is difficult to diagnose by presence of symptoms alone. The overall sensitivity of the LFT strips was 73.3% (Table 4). This percentage is higher than any other diagnostic kit response of early stage of infection as reported by Effler *et al*., (2002).

### Leptospira isolation from infected animals

Culture of blood is the most frequent means of diagnosing leptospiremia. Analyses of *Leptospira* isolation revealed that leptospires identification is proof of infection. In this study, use of blood culture in EMJH medium for the leptospires presence in the blood is compared with the relative sensitivity, specificity and efficiency of LFT strips. All infected animal blood samples were inoculated into the EMJH medium, incubated aerobically and protected from light, at room temperature (25°-30°C) for two months, and examined weekly for 45 days, then every five days for a further 30 days. Examination of the cultures was done by placing one drop of culture onto a microscopic glass slide and observe under dark-field microscopy at 200x magnification. The results are considered positive if leptospires are observed under the darkfield microscopy (Table 6).

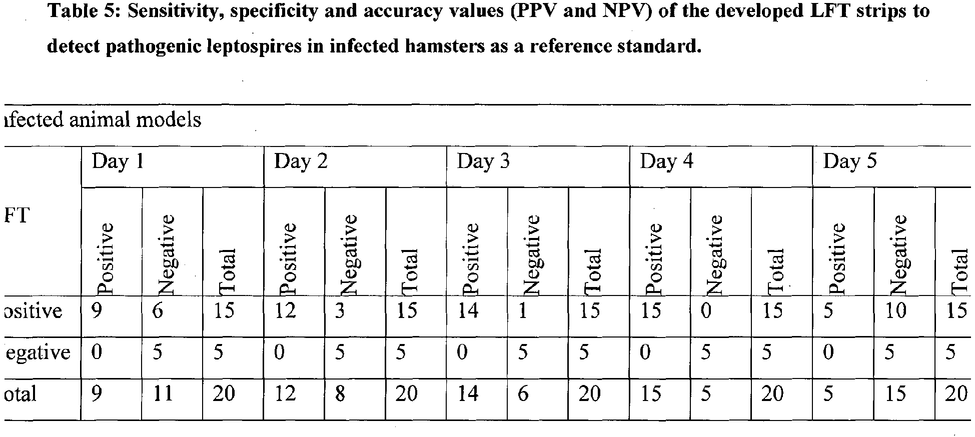

### Detection of leptospires (or DNA) in blood using lipL21 gene specific primer-based PCR

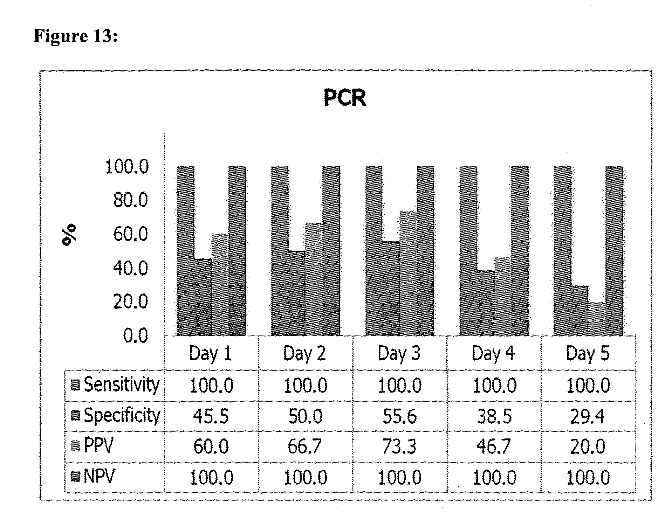

Since the culture of *Leptospira* is difficult and not done in most of the research and clinical laboratories, determination of this bacterium in blood has been widely performed using the PCR technique even though there is no standardized PCR method for detection of *Leptospira.* PCR analysis of leptospirosis, which was considered as one of confirmatory methods for identification and quantification of leptospires, was performed with fifteen infected hamster animal models in parallel with the LFT strips. The observed results were conformed to three repeated tests. The PCR assay using infected animal blood samples was done with lipL21 gene specific primers. An amplified leptospiral DNA was found in some infected animal models (Table 7). The results of PCR in the detection of leptospires in blood samples was considered as positive when the amplified product was observed on agarose gel visualized under UV light (Figure 13).

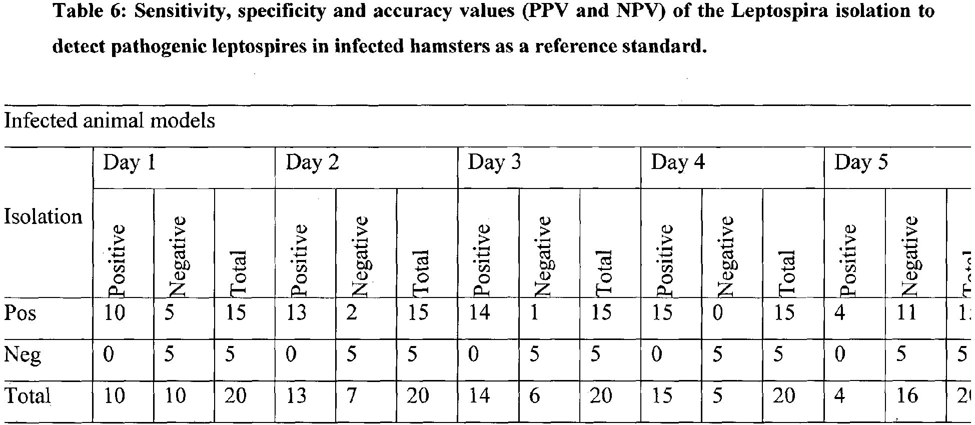

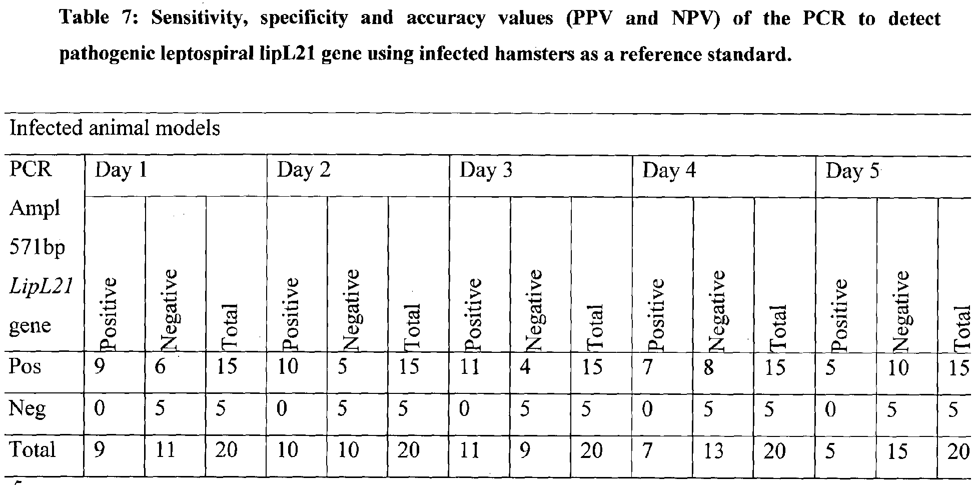

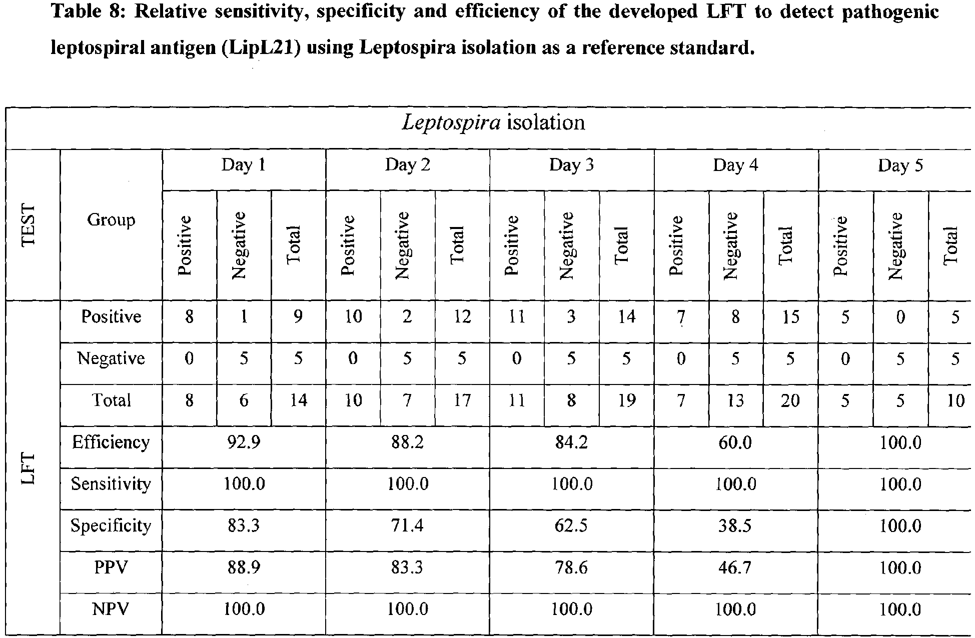

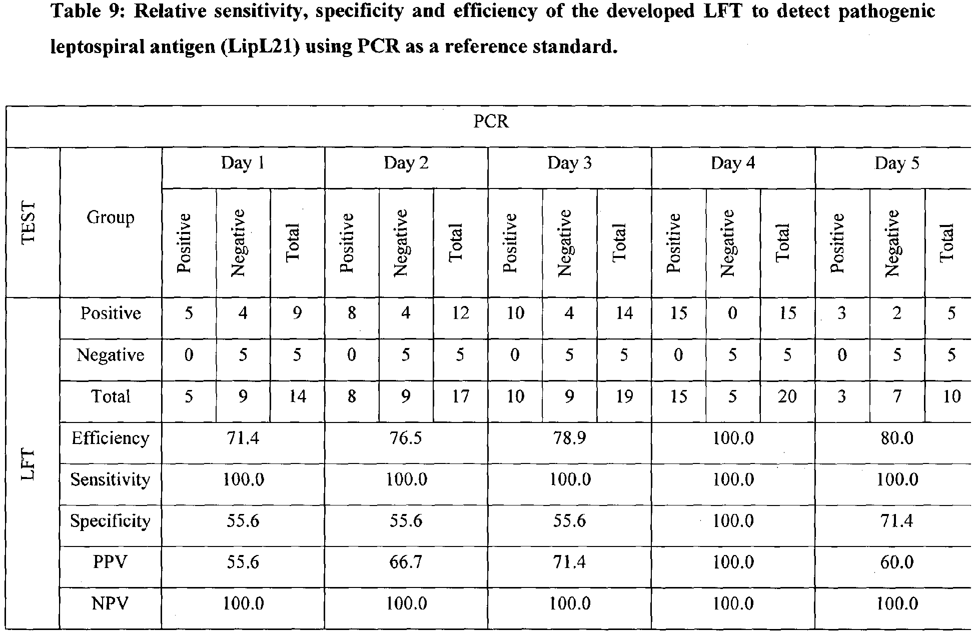

### Sensitivity, specificity and accuracy values (PPV and NPV) and efficiency of LFT strips

An evaluation of LFT strip method for the diagnosis of acute leptospirosis was undertaken by comparison with laboratory-produced techniques employing *Leptospira* isolation and PCR on leptospires infected animal model. Culturing *Leptospira* is the traditional method for isolation and identification of leptospires. The results of LFT and PCR in the detection of leptospires from infected animal model were compared to the growth of leptospires in EMJH semi-solid medium. Results were either positive or negative leptospiral infection and were observed on Day 1, Day 2, Day 3, Day 4 and Day 5 (Table 4).

The LFT strips were compared with the other tests: *Leptospira* isolation and PCR. The LFT strips did not show any false positive results. From these results, the sensitivity and specificity of LFT strips were compared with the other tests (Table 8 and Table 9).

The performance of LFT versus culture isolation is shown in Table 8. Fifteen samples from infected animal models were positive for *Leptospira* on isolation by cultures and were positive by LFT. Compared with culture isolation, sensitivities for LFT were 100% on Day 1 to Day 5. All samples negative on *Leptospira* cultures were negative by LFT for 5 days. However, specificity was 83.3% on Day 1, 71.4 % on Day 2, 62.5% on Day 3, 38.5% on Day 4, and 100% on Day 5. The PPV, NPV and efficiency were all significantly higher when compared with the respective values of PCR (Table 9). In all 15 samples that showed positive reaction for *Leptospira* by LFT strips were also positive by blood culture was for leptospirosis. Furthermore, the *Leptospira* infection not detected by PCR, not all had low leptospiremia. The PCR application to blood samples has many potential drawbacks due to the susceptibility of PCR to inhibitors, contamination and experimental conditions. For instance, it is known that the sensitivity and specificity of a PCR assay is dependent on target genes, primer sequences, PCR techniques, DNA extraction procedures, and PCR product detection methods.

The result of *Leptospira* isolation in the detection of leptospires in animal models was considered as positive when the leptospires were visualized under dark field microscope. The relative sensitivity, specificity and predictive values of LFT strips with *Leptospira* isolation were compared for Day 1-5 post infection (Table 5 and Figure 12). The sensitivity of LFT strips increased from 60 % in the first day to 100 % during next 3-4 days. Figure 13 shows the positivity rates (sensitivity) of lateral flow at different durations post infection: it was 60 % on Day 1, 80 % on Day 2, 93.3 % on Day 3, 100 % on Day 4 and 33.3 % on Day 5. The test was done to detect leptospires on the same day of collection of the blood samples. The specificity did not show large variations between Day 2-4. LFT sensitivity decreased to 33.3 % on the Day 5. The high sensitivity results were observed on Day 1-4 after infection of the hamster model. The high agreement between LFT and culture methods suggests that LFT strips can be particularly important in acute leptospirosis where there were clinical signs and symptoms but absence of antibodies (negative serological results), and thereby allowing early and rapid confirmation of the disease.

There were also significant discrepancies between antigen detection in LFT and between *Leptospira* isolation. Hence, the LFT strip can detect early leptospiral infection in hamsters. Notably, the positive rates of the detection of leptospires in this experimental model gradually declined with time. On Day 5 post infection, this may reflect specific antibody-mediated leptospires clearance to respond to the humoral immunity (Guerreiro *et al*., 2001).

### Reproducibility of LFT strips detection method

*Leptospira* isolation and PCR analysis, which were considered as important confirmatory methods for identification of leptospires, were performed to identify the leptospires from 15 *Leptospira* infected animal models in parallel with the LFT strips.

In all countries except the US & Malaysia a patent application must be filed before the first public disclosure or offer for sale of the invention. However in US & Malaysia, a patent application may be filed within one year of the first public disclosure or offer for sale of the invention

## Claims

1. An *in-vitro* method for the detection of the presence of *Leptospira* in a sample, comprising the steps of
a) providing a sample to be analyzed obtained from a subject suspected to suffer from a *Leptospira* infection between day 1 and day 7 of the infection,
b) detecting the presence of LipL21 in the said sample by use of a polyclonal rabbit anti recombinant-LipL21 (rLipL21) IgG antibody specific for the LipL21 protein,
wherein the presence of LipL21 in the said sample is indicative for the presence of *Leptospira.*

2. The method according to claim 12, further comprising a step of absorbing excess sample fluid in an absorbance zone.

3. The method according to Claim 1 or 2, wherein the sample is a biopsy or a liquid sample.

4. The method according to Claim 3, wherein the subject suspected to suffer from a *Leptospira* infection is a bird, amphibian, reptile or mammal.

5. The method according to any one of Claims 1 to 4, wherein the detection in step b) is performed using a lateral flow test (LFT).

6. A diagnostic device for detecting the presence of *Leptospira* in a sample, the diagnostic device comprises:
a) a sample addition zone,
b) a conjugation zone, comprising an antibody-indicator complex composed of a polyclonal rabbit anti-recombinant LipL21 IgG antibody conjugated to an indicator,
c) a detection zone, comprising a zone of immobilized detection antibodies (capture line) and a control zone of immobilized control antibodies.

7. The diagnostic device according to Claim 6, wherein zones a) to c) are arranged successively on a strip from proximal to distal, wherein the sample fluid can move from zone a) to zone c) by capillary forces.

8. The diagnostic device according to Claim 6 or 7, wherein the indicator is a gold particle.

9. The diagnostic device according to any one of Claim 6 to 8, wherein the detection antibodies are anti-LipL21 antibodies.

10. The diagnostic device according to any one of Claim 6 to 9, wherein the control antibodies are anti-rabbit antibodies.

11. The diagnostic device according to any one of Claim 6 to 10, wherein the zone of immobilized detection antibodies (capture line)contains the antibody in a concentration of between 500µg/ml to 10000µg/ml, and/or wherein the zone of immobilized detection antibodies (capture line) contains 5µg of rabbit anti-LipL21 antibody and/or wherein the control zone contains 1µg of goat anti-rabbit antibody.

12. An *in-vitro* method for diagnosing a *Leptospira* infection, the method comprising the steps of providing a sample obtained from a subject to be diagnosed, adding the sample to a diagnostic device according to any one of claims 6 to 11, wherein the visual appearance of the capture line is indicative for a *Leptospira* infection.

13. Use of a diagnostic device according to any one of Claim 6 to 11, for the diagnosis of leptospirosis, according to the method of any one of claims 1 to 5.

## Patentansprüche

1. Ein In Vitro-Verfahren zum Nachweis der Anwesenheit von *Leptospira* in einer Probe, umfassend die Schritte:
a) Bereitstellen einer zu analysierenden Probe erhalten von einem Subjekt, von dem vermutet wird, an einer Leptospira-Infektion zwischen Tag 1 und Tag 7 der Infektion zu leiden,
b) Nachweisen der Anwesenheit von LipL21 in genannter Probe durch Verwendung eines polyklonalen Kaninchen anti-rekombinantes LipL21 (rLipL21) IgG-Antikörper, der spezifisch ist für einen LipL21-Protein, wobei die Anwesenheit von LipL21 in genannter Probe die Anwesenheit von Leptospira anzeigt.

2. Das Verfahren gemäß Anspruch 12, weiter umfassend ein Schritt zum Absorbieren von überflüssiger Probenflüssigkeit in einer Absorbtionszone.

3. Das Verfahren gemäß Anspruch 1 oder 2, wobei die Probe eine Biopsie oder eine flüssige Probe ist.

4. Das Verfahren gemäß Anspruch 3, wobei das Subjekt, von dem vermutet wird, dass es an einer Leptospira-Infektion leidet ein Vogel, ein Amphibium, ein Reptil oder ein Säuger ist.

5. Das Verfahren gemäß eines der Ansprüche 1-4, wobei der Nachweis in Schritt b) mittels eines lateralen Fluss-Tests (LFT) durchgeführt wird.

6. Ein diagnostisches Gerät zum Nachweis der Anwesenheit von Leptospira in einer Probe, wobei das diagnostische Gerät umfasst:
a) eine Probenadditionszone,
b) eine Konjugationszone, umfassend einen Antikörper-Indikatorkomplex zusammengesetzt aus einem polyklonalen Kaninchen anti-rekombinantes LipL21 igG-Antikörpers konjugiert an einen Indikator,
c) eine Nachweiszone, umfassend eine Zone von immobilisierten Nachweis-Antikörpern (Einfanglinie) und eine Kontrollzone von immobilisierten Kontroll-Antikörpern.

7. Das diagnostische Gerät gemäß Anspruch 6, wobei Zonen a) bis c) nacheinander auf einem Streifen proximal bis distal angeordnet sind, wobei sich die Probenflüssigkeit von Zone a) bis Zone c) durch kapillare Kräfte bewegen kann.

8. Das diagnostische Gerät gemäß Anspruch 6 oder 7, wobei der Indikator ein Goldpartikel ist.

9. Das diagnostische Gerät gemäß eines der Ansprüche 6-8, wobei die Nachweis-Antikörper Anti-LipL21 Antikörper sind.

10. Das diagnostische Gerät gemäß eines der Ansprüche 6-9, wobei die Kontroll-Antikörper Anti-Kaninchen Antikörper sind.

11. Das diagnostische Gerät gemäß eines der Ansprüche 6-10, wobei die Zone der immobilisierten Nachweis-Antikörper (Einfanglinie) den Antikörper in einer Konzentration von zwischen 500 µg/ml bis 10000 µg/ml beinhaltet, und/oder wobei die Zone der immobilisierten Nachweis-Antikörper (Einfanglinie) 5 µg von Kaninchen-Anti-LipL21-Antikörper enthält und/oder wobei die Kontrollzone 1 µg von Ziegen-Anti-Kaninchen-Antikörper enthält.

12. Ein In-vitro-Verfahren zur Diagnose einer Leptospira-Infektion, das Verfahren umfassend die Schritte der Bereitstellung einer Probe abgenommen von einem Subjekt, das diagnostiziert werden soll, Hinzufügen der Probe zu einem diagnostischen Gerät gemäß eines der Ansprüche 6-11, wobei die visuelle Entstehung der Einfanglinie eine Leptospira-Infektion anzeigt.

13. Verwendung eines diagnostischen Geräts gemäß eines der Ansprüche 6-11 zur Diagnose einer Leptospirosis, gemäß einem Verfahren eines der Ansprüche 1-5.

## Revendications

1. Procédé *in vitro* pour la détection de la présence de *Leptospira* dans un échantillon, comprenant les étapes de :
a) la fourniture d'un échantillon à analyser obtenu à partir d'un sujet suspecté de souffrir d'une infection à *Leptospira* entre le jour 1 et le jour 7 de l'infection,
b) la détection de la présence de LipL21 dans ledit échantillon par l'utilisation d'un anticorps polyclonal IgG de lapin anti-LipL21 recombinant (rLipL21) spécifique de la protéine LipL21,
dans lequel la présence de LipL21 dans ledit échantillon indique la présence de *Leptospira.*

2. Procédé selon la revendication 12, comprenant en outre une étape d'absorption de l'excès de liquide de l'échantillon dans une zone d'absorbance.

3. Procédé selon la revendication 1 ou 2, dans lequel l'échantillon est une biopsie ou un échantillon liquide.

4. Procédé selon la revendication 3, dans lequel le sujet suspecté de souffrir d'une infection à *Leptospira* est un oiseau, un amphibien, un reptile ou un mammifère.

5. Procédé selon l'une quelconque des revendications 1 à 4, dans lequel la détection dans l'étape b) est effectuée en utilisant un test à flux latéral (LFT).

6. Dispositif de diagnostic pour détecter la présence de *Leptospira* dans un échantillon, le dispositif de diagnostic comprend :
a) une zone d'ajout de l'échantillon,
b) une zone de conjugaison, comprenant un complexe anticorps-indicateur composé d'un anticorps polyclonal IgG de lapin anti-LipL21 recombinant conjugué à un indicateur,
c) une zone de détection, comprenant une zone d'anticorps de détection immobilisés (ligne de capture) et une zone témoin d'anticorps témoins immobilisés.

7. Dispositif de diagnostic selon la revendication 6, dans lequel les zones a) à c) sont arrangées successivement sur une bandelette de la partie proximale à la partie distale, dans lequel le liquide de l'échantillon peut se déplacer de la zone a) vers la zone c) par des forces capillaires.

8. Dispositif de diagnostic selon la revendication 6 ou 7, dans lequel l'indicateur est une particule d'or.

9. Dispositif de diagnostic selon l'une quelconque des revendications 6 à 8, dans lequel les anticorps de détection sont des anticorps anti-LipL21.

10. Dispositif de diagnostic selon l'une quelconque des revendications 6 à 9, dans lequel les anticorps témoins sont des anticorps anti-lapin.

11. Dispositif de diagnostic selon l'une quelconque des revendications 6 à 10, dans lequel la zone d'anticorps de détection immobilisés (ligne de capture) contient l'anticorps dans une concentration située entre 500 µg/ml et 10 000 µg/ml, et/ou dans lequel la zone d'anticorps de détection immobilisés (ligne de capture) contient 5 µg d'anticorps de lapin anti-LipL21 et/ou dans lequel la zone témoin contient 1 µg d'anticorps de chèvre anti-lapin.

12. Procédé *in vitro* pour le diagnostic d'une infection à *Leptospira,* le procédé comprenant les étapes de la fourniture d'un échantillon obtenu à partir d'un sujet à diagnostiquer, l'ajout de l'échantillon à un dispositif de diagnostic selon l'une quelconque des revendications 6 à 11, dans lequel l'apparition visuelle de la ligne de capture indique une infection à *Leptospira.*

13. Utilisation d'un dispositif de diagnostic selon l'une quelconque des revendications 6 à 11, pour le diagnostic de la leptospirose, selon le procédé de l'une quelconque des revendications 1 à 5.
